Europäisches Patentamt

European Patent Office

Office européen des brevets

(19)

(11) Veröffentlichungsnummer: **0 188 629**
**A1**

# EUROPÄISCHE PATENTANMELDUNG

(12)

veröffentlicht nach Art. 158 Abs. 3 EPÜ

(21) Anmeldenummer: 85903726.9

(22) Anmeldetag: 24.04.85

Daten der zugrundeliegenden internationalen Anmeldung:

(86) Internationale Anmeldenummer:
**PCT/SU 85/00031**

(87) Internationale Veröffentlichungsnummer:
**WO 86/00621 (30.01.86 86/3)**

(51) Int. Cl.⁴: **C 07 K 7/06**, A 61 K 37/02

(30) Priorität: 16.07.84 SU 3772913

(43) Veröffentlichungstag der Anmeldung: 30.07.86
Patentblatt 86/31

(84) Benannte Vertragsstaaten: **CH DE FR GB IT LI SE**

(72) Erfinder: CHAZOV, Evgeny Ivanovich, ul. 3-ya
Cherepkovskaya, 15a, Moscow, 121552 (SU)
Erfinder: SMIRNOV, Vladimir Nikolaevich, Juzhinsky
per., 3-7, Moscow, 103104 (SU)
Erfinder: VINOGRADOV, Valentin Antonovich,
Khoroshevskoe shosse, 34-38, Moscow, 123007 (SU)
Erfinder: POLONSKY, Vladimir Markovich, pr.
Mira, 91-3-386, Moscow, 129085 (SU)
Erfinder: TISCHENKO, Valentina Alexeevna, ul.
Marshala Timoshenko, 38-166, Moscow, 121359 (SU)
Erfinder: TITOV, Mikhail Ivanovich, ul.
Kuntsevskaya, 1/5-368, Moscow, 121351 (SU)
Erfinder: BESPALOVA, Zhanna Dmitrievna, ul.
Kuntsevskaya, 1/5-229, Moscow, 121351 (SU)
Erfinder: PEKELIS, Boris Leonidovich, ul. Sivtsev
Vrazhek, 21-36, Moscow, 121002 (SU)

(71) Anmelder: VSESOJUZNY KARDIOLOGICHESKY
NAUCHNY TSENTR AKADEMII MEDITSINSKIKH NAUK
SSSR, 3-ya Cherepkovskaya, 15a Moscou, 121552 (SU)

(74) Vertreter: von Füner, Alexander, Dr. et al, Patentanwälte
Schiff, von Füner Strehl, Schübel-Hopf, Ebbinghaus,
Finck Mariahilfplatz 2 u. 3, D-8000 München 90 (DE)

(54) HEXAPEPTID.

(57) Hexapeptide having a Phe-Ala-Gly-Phe-Gly-Arg structure and possessing hepatoprotective properties.

EP 0 188 629 A1

HEXAPEPTID

Gebiet der Technik

Die vorliegende Erfindung bezieht sich auf die organische Chemie und betrifft insbesondere ein neues Hexapeptid.

Vorheriger Stand der Technik Zur Behandlung der akuten Virushepatitis, Verschlechterung der chronischen Hepatitis und Leberzirrhose werden heutzutage synthetische Glukokortikoide mit Hormonwirkung wie Prednisolon und andere sehr häufig verwendet. Diese Präparate werden in der Regel eine lange Zeit gegeben und führen zu zahlreichen Komplikationen an verschiedenen Organen und Systemen. Glukokortikoide bewirken eine Atrophie der Nebennierenrinde, eine Erhöhung des arteriellen Blutdruckes, die Entwicklung der Steroiddiabetes, Verfettung, Flüssigkeitsstauung, Elektrolytstörungen, Osteoporoseerscheinungen, pathologische Knochenbrüche. Im Magendarmtrakt führen die Glukokortikoide die Entwicklung von mit Blutungen verbundenen Geschwüren herbei, was den Verlauf der chronischen Lebererkrankungen besonders ungünstig beeinflußt. Die schnelle Absetzung von Glukokortikoiden hat eine akute Nebenniereninsuffizienz zur Folge. Remissionen, bedingt durch Behandlung mit den Präparaten dieser Klasse, sind vorübergehend und kurzzeitig. (Scott J., Physiological, pharmacological and pathological actions of glucocorticoids on the digestive system. Clin. gastroenterol, 1981, v.10, p.627-652).

Bekannt sind Hexapeptide verschiedener Struktur, zum Beispiel Hexapeptid His-D-Trp-Ala-Trp-D-Phe-Lys-$NH_2$ (Bowers CY at al.,Endocrinology, 1984, v. 114, No. 5, p. 1537-1545) oder Hexapeptid Tyr-D-Ala-Gly-Phe-Leu-Arg und andere. (A.V.Valdman, O.S.Medvedev. Teoreticheskie predposylki dlya poiska novykh serdechno-sosudistykh sredstv sredi peptidov. Vestnik Akademii meditsinskikh nauk SSSR, 1982, Nr. 5, S. 14-23).

Eine hepatoprotektive Aktivität dieser Verbindungen ist jedoch unbekannt.

## Offenbarung der Erfindung

Das beanspruchte Hexapeptid ist neu und in der Fachliteratur nicht beschrieben.

Der Erfindung liegt die Aufgabe zugrunde, einen neuen Stoff zu entwickeln - ein Hexapeptid, das eine hohe hepatoprotektive Aktivität besitzt, keine Nebenreaktionen hervorruft und in der Medizin Verwendung finden kann.

Die Aufgabe ist dadurch gelöst worden, daß das neue Hexapeptid erfindungsgemäß folgende Struktur aufweist:

Phe-Ala-Gly-Phe-Gly-Arg.

Das erfindungsgemäße Hexapeptid stellt ein weißes Pulver dar, ist in destilliertem Wasser, physiologischer Lösung, Alkohol gut löslich und in Äther, Äthylazetat, Benzol unlöslich. Der Schmelzpunkt liegt zwischen $154^°$ und $156\ ^°C\ (\alpha)_D^{25} = +8,4$ (mit $0,5\ H_2O$).

### Die beste Ausführungsform der Erfindung

Die hepatoprotektive Wirkung des erfindungsgemäßen Hexapeptids wurde im Vergleich zu Prednisolon an männlichen Mäusen der Linie Balb C untersucht.

Bei Mäusen, welche 12 Stunden lang hungerten, wurde die Entwicklung einer akuten Hepatitis durch D-Galaktosaminhydrogenchlorid hervorgerufen, wobei es verdünnt in 0,1 ml physiologischer Lösung intraperitoneal in einer Dosis von 700 mg/kg injiziert wurde. Die Kontrollgruppe von Tieren bekam intraperitoneal 0,1 ml physiologische Lösung. Den Tieren, die D-Galaktosaminhydrogenchlorid bekamen, machte man sofort nach der Einführung desselben die Injektionen des erfindungsgemäßen Hexapeptids (100 µg/kg), Prednisolons (5 mg/kg) oder der physiologischen Lösung (0,1 ml) subkutan. Die Injektionen der untersuchten Stoffe wurden nach 6 Stunden wiederholt. Jede Gruppe bestand aus 12 Tieren. Man tötete die Mäuse durch Dekapitation, sammelte das Blut und bestimmte die Konzentrationen der Alanintransaminase und der Glutamatdehydrogenase im Blutplasma.

Die statistische Auswertung der gewonnenen Ergebnisse erfolgte nach dem t-Test. Als sicher wurden die Unterschiede bei einem Wertigkeitsniveau von 95 % (falls P< 0,05 ist) angenommen. Die gewonnenen Ergebnisse sind in der Tabelle 1 dargestellt.

Tabelle 1

| lfd. Nr. | Tiergruppen | Konzentration der Alanintrans-aminase in JU/e | Konzentration der Glutamat-dehydrogenase in JU/e |
|---|---|---|---|
| 1. | Kontrolle | $113,6\pm5,5$ | $32,5\pm3,8$ |
| 2. | D-Galaktosaminhy-drogenchlorid + phy-siologische Lösung | $170,8\pm10,1^x$ | $121,6\pm16,0^x$ |
| 3. | D-Galaktosaminhydro-genchlorid + erfin-dungsgemäßes Hexa-peptid | $115,4\pm5,2^{xx}$ | $62,8\pm4,9^{xx}$ |
| 4. | D-Galaktosaminhydro-genchlorid + Predni-solon | $151,2\pm14,3$ | $104,5\pm10,1$ |

x-der Unterschied zwischen Gruppen 1 und 2 ist sicher.

xx-der Unterschied zwischen Gruppen 2 und 3 ist sicher.

Wie aus der Tabelle zu ersehen ist, hat das D-Ga-laktosaminhydrogenchlorid eine erhebliche Konzentrations-erhöhung der untersuchten Fermente im Blutplasma herbeigeführt. Die Konzentration der Alanintransaminase stieg dabei um das 1,5fache und die der Glutamatdehydrogenase mehr als dreifach. Das erfindungsgemäße Hexapeptid blokkierte praktisch völlig die Konzentrationserhöhung der Alanintransaminase und verringerte die Konzentration der Glutamatdehydrogenase um die Hälfte. Bei der Einführung von Prednisolon war die Tendenz zur Minderung der Werte der untersuchten Parameter zu verzeichnen.

Das erfindungsgemäße Hexapeptid wurde ebenfalls an einem experimentellen Modell der akuten Leberschädigung bei männlichen Ratten der Linie Vistar durch Tetrachlorkohlenstoff untersucht. Nach 12-stündigem Hungern führte man den Ratten $CCl_4$ verdünnt in Olivenöl in einer Dosis von 1 mg/kg intraperitoneal ein. Nach der Einführung von $CCl_4$ wurde den Ratten das erfindungsgemäße Hexapeptid in 4

- 4 -

verschiedenen Dosen (10, 30, 100 und 300 µg/kg) injiziert. Die Wiederholungsinjektionen des erfindungsgemäßen Hexapeptids erfolgten nach 6, 24 und 30 Stunden. Anstelle des erfindungsgemäßen Hexapeptids bekam die Kontrollgruppe von Tieren eine physiologische Lösung (0,2 ml). Alle Tiergruppen bestanden aus 13 Ratten. Im Blut untersuchte man die Konzentrationen der Alanintransaminase und Glutamatdehydrogenase. Die gewonnenen Ergebnisse sind in der Tabelle 2 angegeben.

Tabelle 2

| lfd Nr. | Tiergruppen | Konzentration der Alanintrans- aminase in JU/e | Konzentration der Gluta- matdehydro- genase in JU/e |
|---|---|---|---|
| 1. | in-vivo-Test | 64,6±3,3 | 17,2±0,6 |
| 2. | $CCl_4$ | 4349,3±2285,1 | 5619,7±1201,7[x] |
| 3. | $CCl_4$+erfindungsge- mäßes Hexapeptid (10 µg/kg) | 4141,5±1094,8 | 1962,0±494,7 |
| 4. | $CCl_4$+erfindungsgemä- ßes Hexapeptid (30 µg/kg) | 3037,8±580,8 | 1487,3±301,5[xx] |
| 5. | $CCl_4$+erfindungsge- mäßes Hexapeptid (100 µg/kg) | 2339,6±333,4 | 874,3±118,3[xx] |
| 6. | $CCl_4$+erfindungsge- mäßes Hexapeptid (300 µg/kg) | 2110,1±374,5 | 603,3±118,0[xx] |

x - der Unterschied zu der Gruppe 1 ist sicher.

xx - der Unterschied zu der Gruppe 2 ist sicher.

Wie aus der Tabelle 2 zu ersehen ist, führt $CCl_4$ zur sicheren Konzentrationssteigerung der Glutamatde- hydrogenase. Die Konzentrationserhöhung der Alanin- transaminase gilt wegen geringen Beobachtungsvolumens

- 5 -

nicht als statistisch gesichert. Das erfindungsgemäße Hexapeptid setzt die beiden Kennwerte je nach der Dosis herab. Besonders deutlich nimmt das spezifischere Leberenzym Glutamatdehydrogenase ab.

Das erfindungsgemäße Hexapeptid blockierte außerdem abhängig von der Dosis die Letalität der Ratten im Falle der Verwendung von Tetrachlorkohlenstoff. Die gewonnenen Ergebnisse sind in der Tabelle 3 dargestellt. Wie daraus ersichtlich ist, war die Letalität bei der Einführung des erfindungsgemäßen Hexapeptids in einer Dosis von 100 µg/kg und 300 µg/kg überhaupt nicht zu erkennen, während Tetrachlorkohlenstoff zum Tod von 77 % der Tiere führt.

Tabelle 3

| Kennwerte | Dosis des erfindungsgemäßen Hexapeptids in Mg/kg | | | | |
|---|---|---|---|---|---|
| | 0 | 10 | 30 | 100 | 300 |
| Anzahl von Ratten, die nach 48 Stunden am Leben geblieben sind | 3 | 6 | 9 | 13 | 13 |
| Letalität (%) | 77 | 54 | $30^x$ | $0^x$ | $0^x$ |

x - der Unterschied zu der Gruppe 0 ist sicher.

Die statistische Auswertung im Falle der durch Tetrachlorkohlenstoff hervorgerufenen Leberschädigung in Bezug auf die Enzymparameter erfolgt nach dem t-Test und hinsichtlich der Letalität nach dem $x^2$-Test.

In allen Experimenten unter Verwendung des erfindungsgemäßen Hexapeptids waren keine Veränderungen des Blutzellbestands und der hämodynamischen Hauptparameter zu verzeichnen. Die akute Toxizität des erfindungsgemäßen Hexapeptids $LD_{50}$ betrug 120 mg/kg.

Der Vergleich des erfindungsgemäßen Hexapeptids mit Somatostatin zeigt, daß Hexapeptid dem letztgenannten in der Wirksamkeit überlegen ist: die Letalität ist gleich

O gegenüber 20 % bei Somatostatin, die höchstwirksame Dosis ist um das 15fache geringer (100 µg/kg für Hexapeptid und 1,5 mg/kg für Somatostatin).

Bei den verwendeten experimentellen Modellen der akuten Leberschädigung setzte also das erfindungsgemäße Hexapeptid die Blutkonzentration der Glutamatdehydrogenase und Alanintransaminase sicher herab, deren Konzentrationserhöhung für den in der Leber ablaufenden pathologischen Prozeß charakteristisch ist. Dies spricht dafür, daß das erfindungsgemäße Hexapeptid hepatoprotektive Eigenschaften besitzt. Prednisolon, appliziert in einer gewöhnlichen parenteralen Dosis, übt keinen ausgesprochenen Einfluß auf die Fermentkonzentration aus.

Das erfindungsgemäße Hexapeptid übertrifft in seiner Aktivität Prednisolon (appliziert in einer 50fachen Dosis) und Somatostatin (appliziert in einer 15fachen Dosis). Das erfindungsgemäße Hexapeptid Phenylalanyl-Alanyl-Glyzyl-Phenylalanyl-Glyzyl-Arginin wird durch schrittweise Aminosäurenkettenverlängerung um jeweils eine Aminosäure ausgehend von Arginin als freie Base und aktivierten Estern der geschützten Aminosäuren synthetisiert, wobei die Schutzgruppen durch katalytische Hydrogenolyse und azidolytischen Abbau entfernt werden.

Zum besseren Verstehen der vorliegenden Erfindung wird folgendes Beispiel zur Herstellung des erfindungsgemäßen Hexapeptids angeführt.

Beispiel

Man suspendiert 1,58 g (9,07 mM) Arginin in 25 ml Dimethylformamid, setzt der Lösung 3,30 g (9,98 mM) p-Nitrophenylester von Karbobenzoxyglyzin zu, rührt das Gemisch bei Raumtemperatur innerhalb von 24 Stunden um. Man dampft Dimethylformamid ein, löst den Rückstand in 5 ml Methanol auf und gibt 300 ml Äther zu. Der ausgefällte Niederschlag wird abfiltriert, auf dem Filter mit Äther gespült, in einem Vakuumexsikkator getrocknet.

Man erhält 3,09 g (93 %) Karbobenzoxy-Glyzyl-Arginin mit einem Schmelzpunkt von 135 bis 135,5 °C

$(\alpha)_D^{25}$ = +10,3 (mit 1 Dimethylformamid).

Rf[1]= 0,25 (n-Butanol:Essigsäure:Wasser wie 3:1:1) (A)

Rf[2]= 0,54 (Chloroform:Methanol:32%iger Essigsäure wie 60:45:20) (B)

Rf[3]=0,33 (Äthylazetat:Pyridin:Essigsäure:Wasser wie 45:20:5:11) (C).

Man löst 3,09 g (8,46 mM) Karbobenzoxy-Glyzyl-Arginin in 35 ml Trifluoressigsäure auf, leitet durch die gewonnene Lösung einen Strom von trockenem Hydrogenbromid innerhalb einer Stunde. Man dampft das Lösungsmittel ein, gibt 150 ml Äther dem Rückstand zu, filtriert den ausgefällten Niederschlag ab, löst in Wasser auf und behandelt mit dem Ionenaustauscher Amberlite IR A-410 (OH⁻ -Form) bis zum Erzielen einer negativen Reaktion auf Bromionen. Das Harz wird abfiltriert, auf dem Filter mit Methanol und Wasser gewaschen, das Filtrat wird eingedampft und das restliche Wasser durch azeotrope Destillation über Isopropylalkohol entfernt. Den Rückstand löst man in 25 ml Dimethylformamid auf, gibt der Lösung 3,91 g (9,31 mM) p-Nitrophenylester von Karbobenzoxy-Phenylalanin zu. Das Reaktionsgut wird bei Raumtemperatur innerhalb von 24 Stunden gehalten. Man dampft Dimethylformamid ein, löst den Rückstand in 5 ml Methanol und fügt 300 ml Äther hinzu. Der ausgefällte Niederschlag wird abfiltriert, auf dem Filter mit Äther gespült und in einem Vakuumexsikkator getrocknet.

Man erhält 3,75 g (86 %) Karbobenzoxy-Phenylalanyl--Glyzyl-Arginin mit 133 bis 134 °C Schmelzpunkt $(\alpha)_D^{25}$ = 17,2 (mit 1 Dimethylformamid). Rf[1] = 0,30 (A), Rf[2]= 0,66 (B), Rf[3] =0,43 (C).

Karbobenzoxy-Glyzyl-Phenylalanyl-Glyzyl-Arginin wird analog der beschriebenen Herstellung von Karbobenzoxy--Phenylalanyl-Glyzyl-Arginin ausgehend von 2,71 g (5,28 mM) des letztgenannten und 1,93 g (5,83 mM) p-Nitrophenylester von Karbobenzoxy-Glyzin hergestellt.

Man erhält 2,46 g (82 %) Karbobenzoxy-Glyzyl-Phenylalanyl-Glyzyl-Arginin mit 142 bis 144 °C Schmelzpunkt $(\alpha)_D^{25}$ = -17,3 (mit 1 Dimethylformamid). Rf[1]=0,57 (B),

$Rf^2 \doteq 0,35$ (C), $Rf^3 = 0,45$ (n-Butanol:Ameisensäure:Wasser wie 15:3:1).

Ausgehend von 2,46 g (4,32 mM) Karbobenzoxy-Glyzyl-Phenylalanyl-Glyzyl-Arginin und 1,64 g (4,75 mM) p-Nitrophenylester von Karbobenzoxy-Alanin erhält man analog dem oben beschriebenen 2,13 g (77 %) Karbobenzoxy-Alanyl-Glyzyl-Phenylalanyl-Glyzyl-Arginin mit 148 bis 149 °C Schmelzpunkt $(\alpha)_D^{25} = -20,8$ (mit 1 Dimethylformamid). $Rf^1 = 0,64$ (B), $Rf^2 = 0,38$ (C), $Rf^3 = 0,39$ (n-Butanol:Pyridin:Ammoniak konz.:Wasser wie 20:12:3:15) (D).

Ausgehend von 0,64 g (0,99 mM) Karbobenzoxy-Alanyl-Glyzyl-Phenylalanyl-Glyzyl-Arginin und 0,46 g (1,1 mM) p-Nitrophenylester von Karbobenzoxy-Phenylalanin erhält man analog dem beschriebenen 0,63 g (81 %) Karbobenzoxy-Phenylalanyl-Alanyl-Glyzyl-Phenylalanyl-Glyzyl-Arginin mit 150 bis 153 °C Schmelzpunkt $(\alpha)_D^{22} = -3,5$ (mit 1 MeOH). $Rf^1 = 0,42$ (A), $Rf^2 = 0,70$ (B), $Rf^3 = 0,39$ (C).

Man löst 0,63 g (0,80 mM) Karbobenzoxy-Phenylalanyl-Alanyl-Glyzyl-Phenylalanyl-Glyzyl-Arginin in 10 ml Methanol auf und hydriert über einen Palladiumkatalysator. Der Katalysator wird abfiltriert, auf dem Filter mit Methanol gewaschen, eingedampft, der Rückstand wird aus 5 ml Methanol mit Äther (100 ml) umgefällt und in einem Vakuumexsikkator getrocknet. Das erhaltene Produkt wird auf eine mit Sephadex SP C-25 gefüllte Säule (600x15) gegeben und bei einem Gradienten des Pyridin-Azetat-Puffers, das 0,05 bis 1,00 M beträgt, fraktioniert.

Man erhält 0,34 g (65 %) Phenylalanyl-Alanyl-Glyzyl-Phenylalanyl-Glyzyl-Arginin mit 154 bis 156 °C Schmelzpunkt $(\alpha)_D^{25} = +8,4$ (mit 0,5 $H_2O$). $Rf^1 = 0,58$ (B), $Rf^2 = 0,32$ (D), $Rf^3 = 0,44$ (n-Butanol:Pyridin:Essigsäure::Wasser wie 10,5:5:1:7,5).

Analysenbefund für die Aminosäuren Phenylalanin 2,13 (2), Alanin 1,00 (1), Glyzyn 1,97 (2), Arginin 0,93 (1)

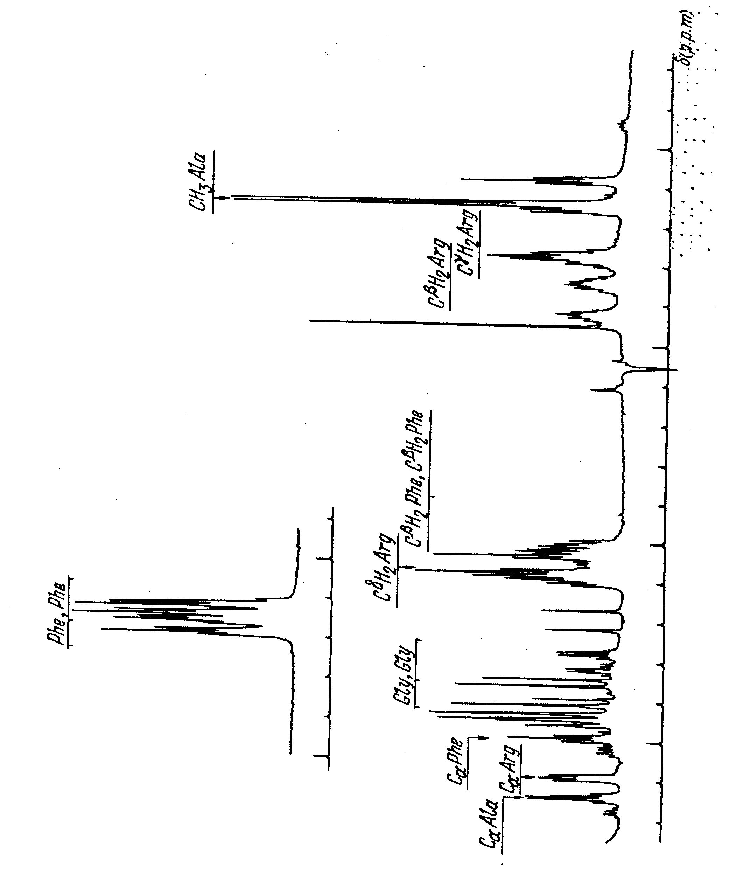

Die Individualität des synthetisierten Hexapeptids wurde mit Hilfe eines NMR-Spektrums (Fig.1) und der Methode der Hochleistungs-Flüssigkeitschromatografie nachgewiesen.

Das Peptid wurde mit 1 Peak bei einem Gradienten von 39,3 % nach 12,8 Minuten eluiert.

(Säule 250x4,6 mm, Spherisorb ODS, 5 µ; mobile Phase A; 0,05 M $KH_2PO_4$; pH 3,0; B: $CH_3CN$; Gradient 20 %→50 % B innerhalb von 20 min; Druck 1500 psi; Geschwindigkeit 1 mm/min; Nachweis bei 214 nm).

Industrielle Anwendbarkeit

Das erfindungsgemäße Hexapeptid besitzt eine hepatoprotektive Wirkung und kann in der Medizin zur Behandlung von akuten und chronischen Lebererkrankungen Verwendung finden.

0188629

PATENTANSPRUCH

Hexapeptid mit folgender Struktur:

Phe-Ala-Gly-Phe-Gly-Arg

Phe, Phe

CH₃ Ala

C^δH₂ Arg

Gly, Gly

C^βH₂ Phe, C^βH₂ Phe

C^βH₂ Arg

C^γH₂ Arg

CₐPhe

CₐAla

CₐArg

δ(p.p.m)

1/1

0188629

# INTERNATIONAL SEARCH REPORT

0188629

International Application No PCT/SU 85/00031

## I. CLASSIFICATION OF SUBJECT MATTER (if several classification symbols apply, indicate all)

According to International Patent Classification (IPC) or to both National Classification and IPC

$IPC^4$ – C 07 K 7/06, A 61 K 37/02

## II. FIELDS SEARCHED

### Minimum Documentation Searched [7]

| Classification System | Classification Symbols |
|---|---|
| $IPC^3$ | C 07 C 103/52, A 61 K 37/02 |

### Documentation Searched other than Minimum Documentation to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | Vestnik Akademii Meditsinskikh Nauk SSSR, No. 5, 1982 (Meditsina Moscow), A.V. Valdman et al "Teoreticheskie predposylkidlya poiska novykh serderno-sosudistykh sredstvsredi peptidov", see pages 14-22 | 1 |
| A | US, A, 4412988 (Roussel Uclaf), 1 November 1983 (01.11.83) | 1 |
| A | EP, A2, 0028716, (HOECHST AKTIENGESELLSCHAFT) 20 May 1981 (20.05.81) | 1 |
| A | WO, A1, 81/00712, (TROPONWERKE GMBH & CO, KG), 19 March 1981 (19.03.81) | 1 |

------

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 7 August 1985 (07.08.85) | 21 October 1985 (21.10.85) |

| International Searching Authority | Signature of Authorized Officer |
|---|---|
| ISA/SU | |